# EUROPÄISCHE PATENTANMELDUNG

(11) **EP 0 992 287 A2**
(43) Veröffentlichungstag der Anmeldung: **12.04.2000**
(21) Anmeldenummer: 99119912.6
(22) Anmeldetag: 08.10.1999
(51) Int. Cl.: B01L 3/00, A61B 5/15, G01N 33/487, G01N 33/49

(54) **Vorrichtung zur Aufnahme und Aufbewahrung einer Substanz für eine Analyse**

(30) Priorität: 08.10.1998 DE 19846466
(71) Anmelder: GHS Gesundheits-Service AG, 74379 Ingersheim (DE)
(72) Erfinder: Baumgärtner, Manfred, Dr., D-70569 Stuttgart (DE); von Bischopinck, Karl Ulrich, D-74379 Ingersheim (DE)
(74) Vertreter: Schwabe - Sandmair - Marx

(57) **Zusammenfassung**

Vorrichtung zur Aufnahme und Aufbewahrung einer Substanz für eine Analyse, die Vorrichtung umfassend:
a) eine Probennahmeeinrichtung (1) mit einem Einlass (2a) zur Aufnahme der Substanz
b) und eine Aufbewahrungseinrichtung (6), die zur Aufbewahrung der Substanz Aufbewahrungskapillaren (7) aufweist, die mit der Probennahmeeinrichtung (1) verbunden sind und in die die aufgenommene fließfähige oder in einen fließfähigen Zustand versetzte Substanz durch kapillaren Hub gefördert wird,
c) wobei die Substanz in den Aufbewahrungskapillaren (7) für eine spätere Analyse bevorrated wird
d) und die Probennahmeeinrichtung (1) und die Aufbewahrungseinrichtung (6) auf oder in einem gemeinsamen Vorratschip (8) ausgebildet sind.

## Beschreibung

Die Erfindung betrifft eine Vorrichtung zur Aufnahme und Aufbewahrung einer Substanz für eine Analyse. Die Erfindung betrifft ferner eine Vorrichtung zur Untersuchung einer Substanz auf wenigstens einen ihrer Inhaltsstoffe. Hierbei kann es sich insbesondere um den Nachweis des Inhaltsstoffs und/oder die Ermittlung der Konzentration des Inhaltsstoffs handeln. In einer Weiterbildung handelt es sich bei der Untersuchung um den Nachweis von mehreren unterschiedlichen Inhaltsstoffen simultan und/oder die Ermittlung der Konzentrationen der mehreren Inhaltsstoffe.

Die Gesundheitsüberwachung im Rahmen der Prophylaxe von Zivilisationskrankheiten und Gesundheitsrisiken gewinnt immer mehr an Bedeutung. Dazu ist die effiziente und kostensparende Untersuchung von Blut- und/oder Urinparametern unerlässlich. Bisher beruht die Überwachung auf Blutprobennahmen beim Arzt oder Apotheker. Es wird venöses oder Kapillarblut entnommen, das ins Labor versandt oder auf sogenannten POC (Point of Care)-Analysatoren untersucht wird.

Im Falle einer venösen Blutprobennahme ist Fachpersonal, beispielsweise ein Arzt, zur Probennahme nötig. Da das Blut bei Verwendung von POC-Analysatoren auf unterschiedliche Teststäbchen aufgebracht wird und diese nacheinander analysiert werden müssen, ergibt sich auch hierfür ein erheblicher Zeit- und Bedienungsaufwand durch geübtes Fachpersonal.

Durch diese Limitierungen gelingt es im Moment nicht, an sich medizinisch wünschenswerte Vorsorgeuntersuchungen flächendeckend durchzusetzen. Wünschenswert für eine breite Akzeptanz sind preiswerte Geräte, die vom Patienten selbst bedient werden können. Dabei sollte eine Blutprobennahme so weit als möglich schmerzfrei und ohne das Austreten von Blut genommen werden können.

Es ist eine Aufgabe der Erfindung, eine preiswerte Vorrichtung zu schaffen, mit der ungeschulte Personen eine Probe einer stofflich zu untersuchenden Substanz aufnehmen können und die eine standardisierte Untersuchung der Probe erleichtert.

Diese Aufgabe wird durch die Gegenstände der unabhängigen Ansprüche gelöst.

In einer besonders bevorzugten Ausführung der Erfindung werden eine Probennahmeeinrichtung und eine kapillare Aufbewahrungseinrichtung auf einem einzigen Trägerchip gemeinsam ausgebildet. Hierbei weist die Probennahmeeinrichtung einen Einlass zur Aufnahme einer Substanz auf, die einer späteren stofflichen Analyse unterzogen werden soll. Die Aufbewahrungseinrichtung weist Aufbewahrungskapillaren auf Die aufgenommene Substanz wird durch kapillaren Hub aus der Probennahmeeinrichtung in die Aufbewahrungskapillaren gefördert. Soweit es sich bei der Substanz nicht von vorherein um eine Flüssigkeit handelt, wird die Substanz noch in der Probennahmeeinrichtung in einen fließfähigen, kapillar förderbaren Zustand versetzt. Die Substanz wird vorzugsweise durch eine Körperflüssigkeit gebildet, insbesondere Blut oder Urin. Die Substanz kann auch ein Umweltmedium sein, insbesondere Wasser, von dem eine Probe aus der Umwelt genommen wird, um beispielsweise die Trinkwasserqualität zu prüfen. Die Substanz kann ferner auch von fester Konsistenz sein, wird dann jedoch vor der Förderung in die Aufbewahrungskapillaren in einer Flüssigkeit gelöst, suspendiert oder verdünnt. Die Probennahme- und Aufbewahrungseinrichtung auf einem gemeinsamen Trägerchip wird nachfolgend auch als Vorratschip bezeichnet.

Mit der Probennahme- und Aufbewahrungseinrichtung separat auf einem eigenen Trägerchip, hergestellt vorzugsweise in Mikrosystemtechnik, wird Blutplasma schmerzfrei, vorzugsweise am Ohrläppchen oder am Finger aus Kapillarblut gewonnen.

Das Probenahmeteil wird in einem bevorzugten Ausführungsbeispiel sodann mit einer Chipkarte verbunden, vorzugsweise in diese eingelegt, auf der eine Reaktoreinrichtung ausgebildet ist. Die Reaktoreinrichtung ist vorzugsweise ebenfalls kapillar ausgeführt, insbesondere in Mikrosystemtechnik. Als Reaktorträger dient vorzugsweise ein Mikrochip aus Silizium oder einem Polymerwerkstoff. Weiterhin sind auf der Chipkarte sämtliche Personeninformationen für die Auswertung und Detektion vorzugsweise als Magnetstreifen angebracht. Die Chipkarte wird in ein Detektions- und Auswertegerät eingeschoben. Dort werden die in der Reaktoreinrichtung gebildeten Reaktionsprodukte detektiert, die Personeninformationen gelesen, miteinander verrechnet und am Display angezeigt. Die Daten können über eine Schnittstelle an einen PC übergeben werden. Die Untersuchung kann vom Verwender selbst durchgeführt werden. Der Vorratschip vereinfacht die Probennahme und gewährleistet aufgrund der Aufbewahrungskapillaren eine besonders gute Reproduzierbarkeit der Messung.

Durch den erfindungsgemäßen Vorratschip mit Aufbewahrungskapillaren und einer integrierten Probennahmeeinrichtung wird ein in Massenfertigung preiswert herstellbarer Artikel geschaffen. Der Verwender erhält eine preiswerte Vorrichtung, mit der er eine Probe selbst nehmen und selbst untersuchen oder an ein Labor für eine professionelle, stoffliche Analyse senden kann. Für die Analyse stehen in dieser Verwendung des Vorratschips sämtliche Möglichkeiten eines analytischen Labors zur Verfügung. Die Kombination aus dezentraler Probennahme unmittelbar beim einzelnen Endverwender und zentraler Analyse hilft bei der Einsparung von Kosten für die breite Überwachung und gestattet dennoch eine umfangreiche Untersuchung.

Zur Probennahme sind die Probennahmeeinrichtung und die Aufbewahrungseinrichtung auf einer von wenigstens zwei Klemmbacken einer Klemmhalterung befestigt oder befestigbar, und die Klemmhalterung ist in der Art einer Wäscheklammer ausgebildet mit einem Rückstellelement, wobei die wenigstens zwei Klemmbacken gegen eine elastische Rückstellkraft des Rückstellelements voneinander abspreizbar sind.

Bei der Kapillarität der Aufbewahrungskapillaren handelt es sich vorzugsweise nicht um eine statistische Materialeigenschaft wie beispielsweise die Porenkapillarität bei porösen Materialien. Der Vorratschip kann gleich bei seiner Herstellung mit den Aufbewahrungskapillaren aufgebaut, insbesondere schichtweise aufgebaut werden. Die Aufbewahrungskapillaren können auch nachträglich eingearbeitet werden. Indem die Aufbewahrungskapillaren je in einer definierten Länge mit einem definierten Querschnitt ausgeführt werden, ist bei vollständiger Füllung auch das Volumen der in je einer der Aufbewahrungskapillaren enthaltenen Flüssigkeit definiert. Dies ist von Vorteil bei der Entleerung des Vorratschips und für die Anlayse der aufbewahrten Substanz. So kann der Inhalt jeder Aufbewahrungskapillare oder von mehreren Aufbewahrungskapillaren maschinell in stets definierter Menge bzw. Volumen in Probenbehältnisse gefüllt und einer Analyse zugeführt werden. Statt einer Umfüllung in Probenbehältnisse kann, wie nachfolgend beschrieben, durch die Ausbildung von je einzeln definierten Aufbewahrungskapillaren der volumenmäßig definierte Inhalt jeder der Kapillaren und damit eine definierte Menge der aufgenommenen Substanz in einer nachfolgenden Reaktoreinrichtung in eindeutiger Zuordnung zu Reaktorkapillaren einer Reaktion zugeführt werden, die für den wenigstens einen Inhaltsstoff oder mehrere Inhaltsstoffe spezifisch ist.

Der Vorratschip ist vorzugsweise als Mikrochip ausgeführt, vorzugsweise aus Silizium oder als polymerer Kunststoff in Mikrospritzguss.

Die Aufbewahrungskapillaren sind vorzugsweise als voneinander separierte Mikrokanäle ausgebildet, zwischen denen eine Fluidverbindung nicht oder nur an Kapillareneinlässen besteht. Obgleich weniger bevorzugt, können die Aufbewahrungskapillaren auch verzweigt sein, wobei jedoch je zwei verzweigte Kapillaren wieder voneinander separate Kapillaren sind. Die Aufbewahrungskapillaren münden vorzugsweise an einer Stirnfläche des Vorratschips in Kapillarenauslässen. An dieser Stirnfläche werden die Auslässe einzeln pro Aufbewahrungskapillare gebildet. In einem bevorzugten Ausführungsbeispiel weisen die Aufbewahrungskapillaren einen gemeinsamen Einlass auf, von dem aus sie zunächst fächerförmig auseinander laufen und dann vorzugsweise parallel nebeneinander bis zu ihren Auslässen sich erstrecken. Der gemeinsame Einlass ist mit einem Auslass oder den Auslässen der Probennahmeeinrichtung verbunden.

Bei der Probennahme erfolgt die Förderung der Substanz durch die Aufbewahrungskapillaren hindurch vorzugsweise nur aufgrund des kapillaren Hubs. In einem bevorzugten Ausführungsbeispiel, in dem auch die Probennahmeeinrichtung durch ein Kapillarsystem gebildet wird, insbesondere ein Kapillarenbündel, erfolgt die gesamte Probennahme bis zur Füllung des Vorratschips mittels kapillaren Hubs. Aktive Systeme zur Förderung der Substanz sind vorteilhafterweise nicht erforderlich, können jedoch unterstützend vorgesehen sein.

Allerdings ist in bevorzugten Ausführungsbeispielen auf dem Vorratschip ein Pulsator angeordnet, der in einem Betriebszustand, vorzugsweise in einem energielosen Ruhezustand, die Aufbewahrungskapillaren einlassseitig fluiddicht verschliesst, so dass nicht unabsichtlich Flüssigkeiten angesogen werden können. Ferner wird eine zusätzliche Sicherheit gegen Entleerung geschaffen. Die Ausbildung eines Pulsators auf dem Vorratschip hat den weiteren Vorteil, dass mit solch einem Pulsator die Strömung für eine kurzzeitiges Spülen der Probennahmeeinrichtung umgekehrt werden kann. Ferner kann der Pulsator zur Spülung einer Reaktoreinrichtung und einer Detektionseinrichtung verwendet werden, insbesondere wenn die Untersuchung der aufgenommenen Substanz vom Verwender zu Hause selbst durchgeführt wird.

Obgleich aufgrund der Ausbildung als Kapillaren bis zum Auslass auch an der Auslassseite des Vorratschips Flüssigkeit nicht austreten kann, sind die Auslässe der Aufbewahrungskapillaren in einem bevorzugten Ausführungsbeispiel luftdurchlässig, aber fluiddicht verschlossen, vorzugsweise durch eine fluiddichte und luftdurchlässige Schutzfolie, die die auslassseitige Stirnfläche des Vorratschips bedeckt. Sie können auch vor einem Versand durch eine Folie fluiddicht verschlossen werden. Das Verschließen dient in erster Linie als Verdunstungsschutz, da ein Auslaufen wegen der Kapillarität nicht möglich ist.

In einer besonders bevorzugten Ausführungsform ist auf dem Vorratschip eine Sollbruchstelle durch Materialschwächung ausgebildet. An der Sollbruchstelle, die vorzugsweise als Sollbruchlinie quer zu den Aufbewahrungskapillaren ausgebildet ist, kann der Vorratschip einfach durchgebrochen werden, um die Probennahmeeinrichtung von den Aufbewahrungskapillaren zu trennen. Durch das Abtrennen der Probennahmeeinrichtung wird die standardisierte Spülung des verbleibenden Teils des Vorratschips erleichtert. Die Aufbewahrungskapillaren sind zwischen der Sollbruchstelle und ihren Auslässen wesentlich länger als zwischen der Sollbruchstelle und der Probennahmeeinrichtung. Besonders bevorzugt verbleibt durch das Abbrechen ein Chipteil, über dessen volle Länge sich die Aufbewahrungskapillaren zwischen ihren Auslässen und den entlang der Sollbruchlinie neu gebildeten Einlässen von einer Stirnfläche zur gegenüberliegenden Stirnfläche erstrecken.

Die Probennahmeeinrichtung wird vorzugsweise durch wenigstens eine Kapillare und besonders bevorzugt durch ein Kapillarenbündel gebildet, die bzw. dass bevorzugt eine nadelförmige Spitze ausbildet. Mit dieser Probennahmeeinrichtung ist eine subkutane Entnahme einer Körperflüssigkeit, insbesondere von Blut, weitestgehend schmerzfrei möglich. Ebenso kann mit dieser Probennahmeeinrichtung jedoch auch eine Flüssigkeitsprobe von einer Flüssigkeit mit einer freien Flüssigkeitsoberfläche aufgenommen werden.

In einer alternativen Ausführungsform, in der die Probennahmeeinrichtung ebenfalls zur Aufnahme von Blut, Urin oder einem flüssigen Umweltmedium wie Wasser dient, kann die Probennahmeeinrichtung durch eine poröses, saugfähiges Material oder durch einen Hohlraum gebildet werden, der mit solchem Material gefüllt ist. Das poröse, saugfähige Material saugt die Flüssigkeit auf und filtert sie vor der Übergabe an die Aufbewahrungskapillaren. In einer weiteren alternativen Ausführungsform weist die Probennahmeeinrichtung einen Hohlraum auf, der Seitenwände besitzt, die ganz oder zumindest teilweise aus einem porösen Material bestehen. In dem Hohlraum kann festes Probenmaterial, beispielsweise Staub, Bodenproben oder Stuhl aufgenommen werden.

Der Vorratschip ist mit einer Reaktoreinrichtung verbindbar, die separate Reaktorkapillaren aufweist, zwischen denen vorzugsweise keine Fluidverbindungen bestehen. Bei einer Verbindung des Vorratschips mit der Reaktoreinrichtung wird vorzugsweise je eine Aufbewahrungskapillare mit einer Reaktorkapillare verbunden. Die Förderung der Flüssigkeit aus den Aufbewahrungskapillaren in die Reaktorkapillaren erfolgt vorzugsweise mittels kapillaren Hubs. Die Förderung kann jedoch stattdessen oder unterstützend durch Einpumpen einer Transportflüssigkeit oder eines Eluats in die Aufbewahrungskapillaren erfolgen. Zu diesem Zweck ist vorzugsweise auf dem Vorratschip wenigstens ein Spülkanal ausgebildet, der mit einem Flüssigkeitsreservoir verbindbar ist. Der Spülkanal steht mit den Aufbewahrungskapillaren ständig in Verbindung oder ist mit diesen Kapillaren verbindbar und ist vorzugsweise ebenfalls als Kapillare ausgebildet.

Die Reaktoreinrichtung ist an ihrem Auslass vorzugsweise mit einer Detektionseinrichtung verbindbar oder verbunden. Auch die Detektionseinrichtung weist vorzugsweise separate Kapillaren auf, zwischen denen keine Fluidverbindungen bestehen. Diese Detektorkapillaren und die Reaktorkapillaren sind im verbundenen Zustand der Reaktoreinrichtung und der Detektionseinrichtung einzeln dicht miteinander verbunden. In den Kapillaren der Detektionseinrichtung sind je wenigstens ein Detektor, vorzugsweise je mehrere Detektoren, angeordnet.

Mehrere Reaktorkapillaren sind vorzugsweise unterschiedlich auf den zu analysierenden Inhaltsstoffzugeschnitten, wobei die Oberflächen der Reaktorkapillaren mit unterschiedlichen Reagenzien belegt sein können. Es können insbesondere mit Reagenzien belegte Mikrokörper eingebracht sein. Um physikalische Eigenschaften der Flüssigkeit in den Reaktorkapillaren messen zu können, kann es auch vorteilhaft sein, Verzögerungsschleifen und/oder Verzweigungen in den Reaktorkapillaren vorzusehen, um beispielsweise gezielt Strömungswiderstände zu erzeugen. Besonders bevorzugt sind Reaktorkapillaren in solch einer Anzahl vorgesehen, dass parallel mehrere Inhaltsstoffe spezifische Reaktionen eingehen, anhand deren sie detektiert und/oder ihre jeweilige Konzentration ermittelt werden kann. Es können daher in besonders bevorzugten Ausführungsbeispielen mehrere Untersuchungsmethoden für ein und denselben Inhaltsstoff zum Einsatz kommen und dies für mehrere Inhaltsstoffe gleichzeitig. Dies erlaubt eine umfassende Untersuchung der aufgenommenen Substanz.

Die Erfindung betrifft ferner eine Vorrichtung zur Untersuchung einer Substanz. Die Untersuchung besteht bevorzugt in einem Nachweis wenigstens eines Inhaltsstoffs der Substanz und/oder der Messung der Konzentration des Inhaltsstoffs in der Substanz. Die Vorrichtung umfasst eine Probennahmeeiririchtung, eine Aufbewahrungseinrichtung, eine Reaktoreinrichtung und eine Detektionseinrichtung, die je die vorstehend bereits genannten Funktionen erfüllen und vorzugsweise wie vorstehend beschrieben ausgebildet sind. Allerdings müssen die Probennahmeeinrichtung und die Aufbewahrungseinrichtung nicht unumgänglich auf einem eigens hierfür vorgesehenen Vorratschip ausgebildet sein. Die Probennahmeeinrichtung, die Aufbewahrungseinrichtung, die Reaktoreinrichtung und auch die Detektionseinrichtung und vorzugsweise auch noch eine Auswerteeinrichtung können grundsätzlich auch zusammen auf einem einzigen gemeinsamen Träger angeordnet sein. Besonders bevorzugt wird jedoch die Ausbildung eines Vorratschips nur mit der Probennahmeeinrichtung und der Aufbewahrungseinrichtung.

In einem bevorzugten modularen Aufbau ist die Reaktoreinrichtung auf einem eigenen Reaktorchip vorgesehen. Der Reaktorchip ist desweiteren auf einem Träger angeordnet, besonders bevorzugt in eine Chipkarte einlegbar, auf dem vorzugsweise personenbezogene Daten des Probanden gespeichert sind und der vorzugsweise ein Flüssigkeitsreservoir zum Spülen der Reaktoreinrichtung und/oder ein Abfallreservoir aufweist. Das Flüssigkeitsreservoir ist mit einer Elutions- oder Förderflüssigkeit gefüllt. Die Flüssigkeit wird passiv durch kapillaren Hub oder aktiv durch die Aufbewahrungskapillaren und Reaktorkapillaren hindurch gefördert. In dem Abfallreservoir wird die Flüssigkeit nach dem Durchströmen der Reaktoreinrichtung und der Detektionseinrichtung aufgefangen und entsorgt, beispielsweise verdunstet. Durch den modularen Aufbau mit einem eigenen Reaktorchip zusätzlich zum Vorratschip ist eine Gesundheitsüberwachung besondes preiswert durchführbar. Der Reaktorchip ist ebenfalls als Mikrochip gefertigt, vorzugsweise als Siliziumchip oder in Mikrospritzguss aus einem Polymerkunststoff.

Eine weitere erfindungsgemäße Vorrichtung zum Nachweis wenigstens eines Inhaltsstoffs einer Substanz umfasst eine Probennahmeeinrichtung mit einem Einlass zur Aufnahme der Substanz und eine Reaktoreinrichtung. Die Reaktoreinrichtung weist Reaktoren auf, die durch die vorstehend beschriebenen, voneinander separierten Reaktorkapillaren oder durch eine saugfähige Materialschicht gebildet werden. Die Reaktoren sind mit der Probennahmeeinrichtung verbunden. Die mit der Probennahmeeinrichtung aufgenommene, fließfähige oder in einen fließfähigen Zustand versetzte Substanz wird durch kapillaren Hub in die Reaktoren gefördert. Die Reaktoren sind mit wenigstens einem Reagenz belegt, das mit dem nachzuweisenden Inhaltsstoff eine spezifische Farbreaktion eingeht. Die Probennahmeeinrichtung und die Reaktoreinrichtung sind auf einem gemeinsamen Trägerchip ausgebildet.

Diese Vorrichtung erlaubt die Probennahme und den Nachweis wenigstens eines Inhaltsstoffs der Substanz, vorzugsweise Blut, auf einfache Weise durch den Verwender selbst. Eine Aufbewahrungseinrichtung entfällt vorzugsweise. Die Reaktoreinrichtung ist aufgrund der stattfindenen Farbreaktion gleichzeitig die Detektionseinrichtung.

In einem bevorzugten Ausführungsbeispiel wird die Vorrichtung zur Durchführung eines Prostata PSA-Schnelltests verwendet. Dabei handelt es sich um einen qualitativen Schnelltest zum Nachweis des Prostata spezifischen Antigens (PSA) im Vollblut. Die Vorrichtung ermöglicht die preiswerte und selbständige Vorsorgeuntersuchung durch den Verwender.

Konzeptionell baut der mit dieser Vorrichtung durchführbare erfindungsgemäße Schnelltest aufdem sogenannten CARDIMAC-PSA-Test der MEDPRO Ltd., Northampton, UK, auf.

Der Trägerchip ist als Mikrochip aus Silizium oder Polymerkunststoff mit Reaktorkapillaren in Form von separaten Mikrokanälen oder als Plastikstreifen ausgebildet, der mit einer saugfähigen Matrix in Dünnschichchromatographie beschichtet ist.

Bildet eine saugfähige Matrix die Reaktoreinrichtung. so weist die saugfähige Matrix in zwei in Fließrichtung hintereinander angeordneten Zonen B und C immobilisierte PSA Antikörper auf. In einer stromaufwärts von der Zone B angeordneten Zone A ist ein Vliesmaterial, insbesondere ein Vliespapier, auf dem Trägerchip angeordnet, auf das Vollblut oder Serum und ein konjugathaltiges Laufmittel aufgebracht werden. Zur Verstärkung von kapillaren Kräften ist stromabwärts von der Zone C ein weiterer Streifen Vliespapier angebracht. Der Plastikstreifen ist in vorzugsweise ein Kunststoffgehäuse eingesetzt, in dem über den drei Zonen A, B und C jeweils Einsparungen eingearbeitet sind, welche die Durchsicht auf die Zonen A, B und C freigeben. Der Plastikstreifen weist eine Breite von 0,5 ― 2 cm und eine Länge von 4 ― 8 cm auf. Das Kunststoffgehäuse besitzt eine Breite von 2 ― 3 cm und eine Länge von 5 ― 9 cm. Die Aussparungen besitzen je einen Durchmesser von etwa 0,5 cm. Für den vom Verwender selbst durchgeführten Schnelltest wird zunächst ein Tropfen Vollblut auf das Vliesmaterial in Zone A aufgebracht und mit einem mitgelieferten Laufmittel verdünnt. Während das Vliesmaterial die Erythrozyten und andere störende, partikuläre Blutbestandteile zurückhält, fließt das verdünnte Blutserum durch die saugfähige Matrix den Teststreifen entlang zur Zone B und schließlich zur Zone C. Ein in dem Laufmittel vorhandenes Antikörper-Farbkonjugat bildet mit den eventuell im Blut vorhandenen PS-Antigenen einen Antigen-Antikörperkomplex. Nach dem immunochromatographischen Reaktionsprinzip reagiert dieser Komplex in der Zone B mit dem immobilisierten Antikörpern und bildet einen sichtbaren Streifen. In Zone C sind bereits Antigen-Antikörperkomplexe immobilisiert und bilden mit dem Farbkonjugat einen positiven Kontrollstreifen. Der positive Nachweis ist nach einer Inkubationszeit von zehn bis fünfzehn Minuten anhand der Farbreaktion, nämlich der Streifenbildung in den Zonen B und C, abzulesen.

Besonders bevorzugt sind in den Aussparungen zur Ablesung der Farbreaktion Lupengläser eingebracht. Vorzugsweise wird das Ende der Inkubationszeit durch einen eingebauten Timer angezeigt, insbesondere durch ein akustisches Signal. Der Timer wird vorzugsweise durch die manuelle Blutentnahme oder durch einen integrierten Leitfähigkeitsdetektor ausgelöst.

Nach der Erfindung kann die Reaktoreinrichtung oder eine Reaktoreinrichtung, die mit dem erfindungsgemäßen Vorratschip zusammenwirkt, wie bei dem PSA-Schnelltest auf einem immunochromatographischen Reaktionsprinzip beruhen, bei dem ein visueller Nachweis des gesuchten Inhaltsstoffs durch Bildung von Antigen-Antikörper-Farbkonjugatkomplexen ermöglicht wird. Anstatt der saugfähigen Matrix können die Reaktoren besonders bevorzugt durch die vorstehend bereits beschriebenen Reaktorkapillaren gebildet werden. An den Oberflächen der Reaktorkapillaren oder in einem in die Reaktorkapillaren eingebrachten, saugfähigen Material sind in einem bestimmten Bereich oder hintereinander liegenden Bereichen immobilisierte, spezifische Antigene und Antikörper lokalisiert, insbesondere wie vorstehend am Beispiel des PSA-Schnelltests.

Das Vliesspapier der am Beispiel des PSA-Schnelltests beschriebenen Vorrichtung kann auch durch das beschriebene Kapillarenbündel ersetzt werden.

In sämtlichen Vorrichtungen der Erfindung können die entsprechenden Trägerchips oder Teststreifen in einem kompakten Gehäuse geschützt untergebracht sein. Nachfolgend wird ein bevorzugtes Ausführungsbeispiel der Erfindung anhand von Figuren erläutert. Es zeigen:
- Fig. 1: eine erfindungsgemäße Analysevorrichtung,
- Fig. 2: einen erfindungsgemäßen Vorratschip,
- Fig. 3: eine Chipkarte mit einer Reaktoreinrichtung und
- Fig. 4: eine Detektions- und Auswerteeinrichtung und die damit verbindbare Chipkarte mit dem eingelegten Vorratschip.

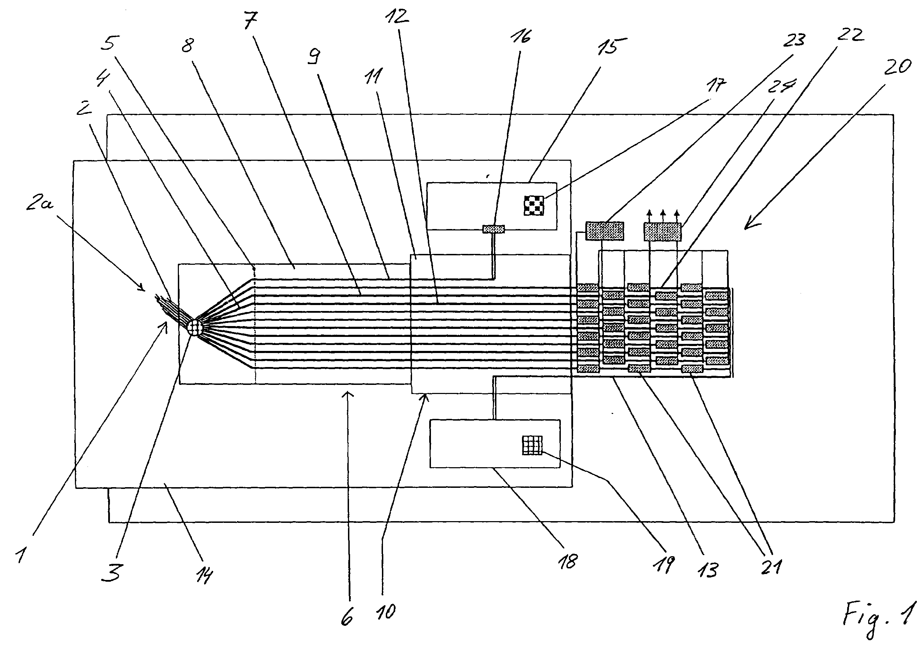

Figur 1 zeigt eine komplette Analysevorrichtung mit einer Probennahmeeinrichtung 1, einer Aufbewahrungseinrichtung 6, einer Reaktoreinrichtung 10, einer Detektionseinrichtung 20 und einer Auswerteeinrichtung 24. Die Analysevorrichtung ist modular aufgebaut, wobei die einzelnen Module in Form von Mikrochips und Chipkarten vorzugsweise aus Silizium oder in Mikrospritzguss aus einem polymeren Kunststoffmaterial hergestellt werden. Die Modularität begünstigt die Standardisierung der Analyse.

Die Module der Analysevorrichtung sind in den Figuren 2 bis 4 einzeln dargestellt. Figur 2 zeigt einen separaten Vorratschip 8, auf dem die Probennahmeeinrichtung 1 und die Aufbewahrungseinrichtung 6 ausgebildet sind. Als Träger für die Probennahmeeinrichtung 1 und die Aufbewahrungseinrichtung 6 dient ein Mikrochip mit einer Breite von höchstens 2 cm, einer Länge von höchstens 3 cm und einer Dicke von höchsten 5 mm. Die Aufbewahrungseinrichtung 6 kann von einem schützenden Gehäuse umgeben sein. Der Vorratschip 8 dient der Aufnahme einer zu analysierenden Substanz und zur Aufbewahrung der aufgenommenen Substanz. Er wird daher als Vorratschip bezeichnet. Er kann insbesondere als Transportmittel für die Substanz zu einem analytischen Labor dienen. Vorzugsweise weist er die in Figur 2 dargestellte Form eines kompakten, dünnen Rechteckblocks auf.

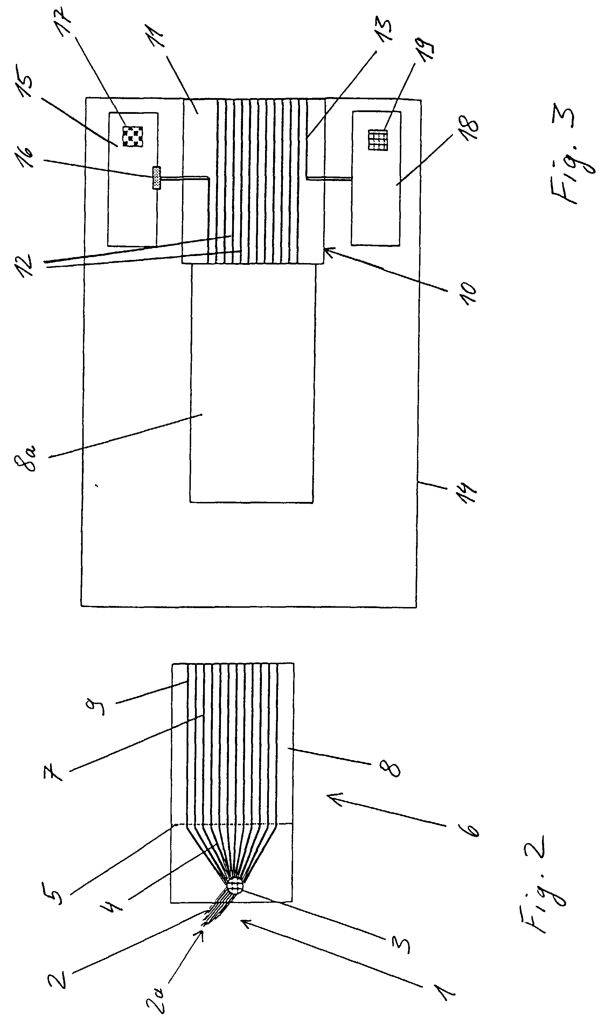

Figur 3 zeigt eine Chipkarte 14, in die ein Reaktorchip 11 eingelegt ist. Aufdem Reaktorchip 11 ist die Reaktoreinrichtung 10 ausgebildet. Die Reaktoreinrichtung 10 könnte anstatt auf einem eigenen Reaktorchip 11 auch integrierter Bestandteil der Chipkarte 14 sein. Ferner sind auf der Chipkarte 14 ein Flüssigkeitsreservoir 15 und ein Abfallreservoir 18 ausgebildet. In Verlängerung der Reaktoreinrichtung 10 stromaufwärts von dieser weist die Chipkarte 14 eine Aussparung 8a auf Der Vorratschip 8 wird für die Analyse in die Aussparung 8a eingelegt, und gleichzeitig wird dabei die Verbindung zwischen der Aufbewahrungseinrichtung 6 und der Reaktoreinrichtung 10 hergestellt. Die beiden Chips 8 und 11 werden auf der Chipkarte 14 gegeneinandergedrückt, so dass an den stumpf aneinanderliegenden Stirnflächen der Chips 8 und 11 die jeweiligen Kapillaren dicht miteinander verbunden werden.

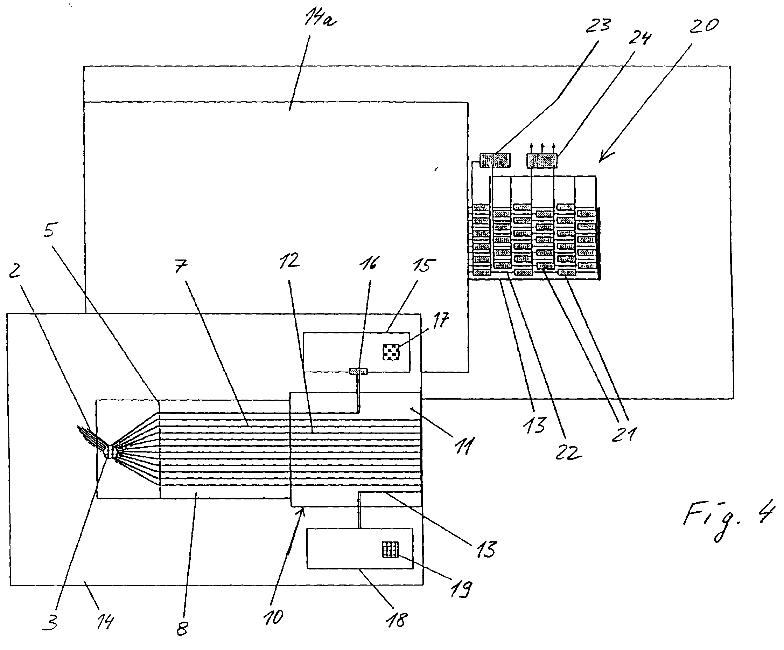

Die Figur 4 zeigt die Chipkarte 14 mit dem eingelegten Vorratschip 8 kurz vor dem Einführen in einen Aufnahmeschacht 14a der Detektions- und Auswerteeinrichtung 20 und 24.

Im folgenden werden die Funktionseinheiten 1, 6, 10, 20 und 24 einzeln und in ihrem Zusammenwirken beschrieben, wofür auf die Figuren 1 bis 4 stets gleichzeitig verwiesen sei.

Eine Blutprobenahme erfolgt mit der Probennahmeeinrichtung 1, die ein Kapillarenbündel mit einer Mehrzahl von Kapillaren 2 aufweist. Die einzelnen Kapillaren 2 sind im Kapillarenbündel dicht nebeneinander angeordnet. Vorzugsweise laufen sie parallel nebeneinander, wie im Ausführungsbeispiel. Sie bilden ein im Querschnitt dicht gepacktes, bevorzugt kreisrundes, Bündel. Die Kapillaren 2 sind in Mikrosystemtechnik aus geeignetem Kunststoff- oder Glasmaterial ausgeführt. Das Kapillarenbündel bildet eine Einstechnadel zum Durchstechen der menschlichen Haut und Eindringen in das unter der Haut liegende Gewebe. Sie ist daher für die subkutane Entnahme einer Körperflüssigkeit, insbesondere von Blut geeignet. Ein Eindringen unter die Haut wird durch die nadelförmige Ausführung mit geschlossener Spitze erleichtert. Grundsätzlich können die Kapillaren 2 an ihren Spitzen auch offen sein. Jede Kapillare 2 kann einzeln eine schräge Spitze aufweisen, oder das Kapillarenbündel bildet insgesamt eine einzige schräge Spitze über den Bündelquerschnitt hinweg.

Die Probennahmeeinrichtung 1 ist so ausgebildet, daß eine Separierung von Blutplasma und Blutkörperchen an der Oberfläche der Kapillaren 2 stattfindet. Die Kapillaren 2 sind dazu beginnend kurz nach der Spitze seitlich perforiert Blut dringt nur durch die Poren dieser Perforation in die Kapillaren 2 ein. Die seitlichen Poren bilden den Einlass 2a. Die Poren sind definiert mit einer Porenweite von höchstens 1 µm ausgeführt, sodass Blutkörperchen nicht eindringen können. In einer Weiterbildung ist die Oberfläche der Kapillaren profiliert, wobei Furchen, insbesondere Mikrorinnen, auf die Poren zulaufen und sich an den Poren vereinigen. Dies dient u.a. zur Vermeidung von Verstopfungen während der Ansaugung von Blutplasma.

Der Vorratschip 8 ist zur Probennahme an einer Klemmhalterung lösbar befestigt, insbesondere eingesteckt und vorzugsweise verrastet. Die Klemmhalterung ist als Klammer ähnlich einer Wäscheklammer ausgebildet. Die Klammer wird vorzugsweise am Ohrläppchen oder am Finger angebracht. Durch den sanften Anpressdruck eines elastischen Rückstellelements der Klemmhalterung wird das Kapillarenbündel in die Haut des Ohrläppchens eingedrückt. Durch kapillaren Hub wird Kapillarblut angesaugt. Entsprechend weit ragt das Kapillarenbündel vor.

Die Probennahmeeinrichtung 1 ist mit einer Aufbewahrungseinrichtung 6 verbunden. Die Probennahmeeinrichtung 1 und die Aufbewahrungseinrichtung 6 sind auf dem Vorratschip 8 in Fließrichtung hintereinander und ineinander übergehend gemeinsam angeordnet. In dem Chipmaterial oder an dessen Oberfläche sind eine Mehrzahl von parallel verlaufenden Aufbewahrungskapillaren 7 eingeätzt, die an einem stromaufwärtigen Ende in Richtung auf einen Punkt zulaufen und sich in diesem Bereich in einem gemeinsamen Einlass vereinigen. Im Bereich des fächerförmigen Bereichs sind die Aufbewahrungskapillaren mit 4 bezeichnet. Genau an dem Punkt bzw. der Stelle der Vereinigung ist das Kapillarenbündel in eine Bohrung des Vorratschips 8 dicht aufgesetzt, sodass es aus der Fläche des Vorratschips 8 aufragt und eine kapillare Verbindung zwischen den zwei Kapillarsystemen 2 und 4, 7 besteht.

Auf dem Vorratschip 8 ist zwischen dem Kapillarenbündel der Probennahmeeinrichtung 1 und den Aufbewahrungskapillaren 4 der Aufbewahrungseinrichtung 6 in Mikrosystemtechnik ein Pulsator 3 ausgebildet, der vorzugsweise mittels Piezotechnik betätigt wird. Als Stromversorgung kann eine Miniaturbatterie, vorzugsweise ein Kondensatorelement, dienen. Der Pulsator 3 wird über einen mechanischen Kontakt in der Klemmhalterung beim Anlegen gestartet. Im abgeschalteten, energielosen Zustand trennt der Pulsator mit einer Pulsatormembran das Kapillarenbündel der Probennahmeeinrichtung 1 von den Aufbewahrungskapillaren 4 und 7.

Die Probenahme erfolgt nach Anlegen der Klemmhalterung und Eindringen des Kapillarenbündels. Durch Kontakt mit einem Blutgefäß wird durch kapillaren Hub Blutplasma angesaugt. Der Saugvorgang erfolgt solange, bis die gesamte kapillare Aufbewahrungseinrichtung 6 gefüllt ist. Eine Verstopfung von Poren in der Probennahmeeinrichtung 1 und das Ansaugen von flexiblen Blutkörperchen werden durch den Pulsator 3 verhindert, der den Ansaugstrom rhythmisch kurzzeitig umkehrt und somit einen Spüleffekt verursacht. Gleichzeitig wird so im Gewebe das Umspülen des gesamten Ansaugfeldes mit Blutplasma gewährleistet und vermieden, daß Interstitialflüssigkeit angesaugt wird.

Die Oberflächen der Kapillaren 2 der Probennahmeeinrichtung 1 können mit Wirkstoffen imprägniert sein, die eine Blutgerinnung unterbinden.

Die Aufbewahrungskapillaren 7 münden an einer seitlichen Stirnfläche des Vorratschips 8 in einer Steckverbindung. Die mündungsseitigen Auslässe der Aufbewahrungskapillaren sind vorzugsweise durch eine luftdurchlässige, aber flüssigkeitsdichte Schutzfolie geschützt.

Zur Analyse von Urin- oder Wasserproben kann das Kapillarenbündel in die entsprechenden Medien getaucht werden. Es kann aber auch eine Modifikation der Probennahmeeinrichtung 1 vorgenommen werden. Dabei werden das Kapillarenbündel und der Pulsator 3 durch ein Aufnahmemedium aus saugfähigem porösen Material ersetzt, beispielsweise aus Silikat oder Glas. Auf dieses wird die Probe durch Eintauchen oder Aufpipettieren aufgebraucht. Dieses System kann auch zur Aufbringung von Blut benutzt werden. Hierbei wird das Blut konventionell durch Lanzette gewonnen. Das saugfähige, poröse Material ist auf dem gleichen Trägerchip wie die Aufbewahrungseinrichtung 6 ausgebildet, so dass auch in diesem Fall eine einfache Handhabung, insbesondere ein Versand zu einem Labor, gewährleistet ist.

Zur Analyse von Stuhl-, Partikel-, Hausstaub-, Bodenproben kann statt des Pulsator-Kapillarenbündels ein Mikrogefäß mit der Aufbewahrungseinrichtung 6 verbunden sein, in das die jeweilige Probe verbracht wird. Nach Befüllung wird das Gefäß mit einem Deckel verschlossen. Die Seitenwände des Gefäßes sind porös ausgeführt. Eine Zuleitungskapillare führt dem Gefäß auf einer Seite Extraktionsflüssigkeit zu, die die Probe eluiert. Das Eluat wird durch die gegenüberliegende poröse Seitenwand des Gefäßes filtriert und den Aufbewahrungskapillaren 4, 7 zugeführt, in denen es wieder für eine spätere Analyse in einen definierten Volumen aufbewahrt bzw. bevorratet wird. Das Gefäß ist auf dem gleichen Trägerchip 8 wie die Aufbewahrungseinrichtung 6 ausgebildet, so dass auch in diesem Fall eine einfache Handhabung, insbesondere ein Versand zu einem Labor, gewährleistet ist.

Die Probennahme- und Aufbewahrungseinrichtungen in ihren jeweiligen Ausformungen finden besonders vorteilhaft Verwendung als Transportbehältnis. Dabei wird der Vorratschip 8 nach Probenahme zur Analyse direkt zu einem analytischen Labor versandt, vorzugsweise mit der Rücksendeverpackung gemäß der US-PS 5,934,549. Dort wird die Schutzfolie am Auslass der Aufbewahrungskapillaren 7 entfernt, das Plasma ausgespült und, gegebenenfalls nach Vorbehandlung und Probenteilung, analysiert.

Zur Erleichterung der Ausspülung in einem Labor ist eine Sollbruchstelle 5 ausgebildet, an der die Aufbewahrungseinrichtung 6 von der Probennahmeeinrichtung 1 getrennt werden kann. Dadurch kann der Ausspülvorgang der Aufbewahrungskapillaren 7 gut automatisiert werden. Die Sollbruchstelle 5 ist eine linienförmige Materialschwächung des Vorratschips 8 quer zu den Kapillaren 7 unmittelbar hinter dem Bereich des Übergangs von den fächerförmig auseinanderlaufenden Kapillaren 4 zu den parallelen Kapillaren 7. Die Abtrennung der Probennahmeeinrichtung erleichtert den Transport. Das Abtrennen kann grundsätzlich aber auch erst im Labor erfolgen. Aufgrund ihrer Kapillarität verliert die Aufbewahrungseinrichtung 6 auch nach dem Abtrennen entlang der Sollbruchlinie Flüssigkeit allenfalls durch Verdunstung. Um dies zu verhindern kann die beim Abtrennen gebildete Stirnfläche für einen Transport mit einer fluiddichten Folie überklebt werden. Grundsätzlich kann der Vorratschip aber auch mit Probennahmeeinrichtung versendet werden und auch im Labor, wie in Figur 1 für eine Untersuchung durch den Verwender selbst dargestellt, mit einer Reaktoreinrichtung 6 und einer Detektions- und Auswerteeinrichtung 20, 24 verbunden werden.

Die zu detektierenden Blutinhaltsstoffe reagieren in der Reaktoreinrichtung 10 zu detektierbaren Verbindungen. Die Reaktoreinrichtung 10 weist Reaktorkapillaren 12 auf, die in Mikrosystemtechnik aufeinem weiteren Trägerchip, dem Reaktorchip 11, aufgebracht sind. Die Reaktorkapillaren 12 münden an zwei gegenüberliegenden Seiten des Reaktorchips 11. Die Reaktoreinrichtung 10 ist auf der Chipkarte 14 aufgebracht, in der auch die eng passende Aussparung 8a für den Vorratschip 8 ausgebildet ist. Durch Einlegen werden die vorzugsweise parallelen Kapillaren 7 und 12 beider Chips 8 und 10 einzeln miteinander fluiddicht verbunden. Die Chipkarte 14 wird dann in ein Lesegerät geschoben, welches eine Steckverbindung für die Auslassseite der Reaktoreinrichtung 10 enthält und dicht mit den Kapillaren 12 der Reaktoreinrichtung 10 abschließt.

Die Reaktorkapillaren 12 sind an ihren Innenwandungen mit Reagenzien belegt, beispielsweise immobilisierte oder nicht immobilisierte Antikörper. Die Reagenzien werden in der Produktion der Reaktoreinrichtung 10 vorzugsweise mit Mikro-Freistrahl-Technologie im nl-Bereich aufgebracht und geeignet immobilisiert, z.B. durch chemische Reaktion oder einfach durch Antrocknen. Die Oberflächen der Reaktorkapillaren 12 können geeignet aufgerauht, mit einer Oxidschicht belegt oder anders geeignet modifiziert sein. Ferner können bereits Mikrokörper definiert oder unspezifisch in der gesamten Reaktoreinrichtung 10 oder in Teilbereichen eingebracht werden.

Die Reaktoren sind auf dem Reaktorchip 11 nur als Kapillaren 12 ausgeführt. Es können jedoch auch andere Strukturen je nach analytischem Bedarf hinzugefügt werden, insbesondere semipermeable Membranen, Verzögerungsschleifen, Verzweigungen und/oder Reagenzien-Reservoirs.

Die Detektionseinrichtung 20 besteht aus einem Satz paralleler Kapillaren 22, zwischen denen keine Fluidverbindung besteht und in denen je hintereinander und/oder parallel Mikrodetektoren 21 vorgesehen sind, z.B. Reflektometer UV-,VIS- oder IR-Detektoren, insbesondere Laser-Fotometer, Radiodetektoren, pH/eV-Elektroden und/oder Biosensoren. Je eine solche Detektorkapillare 22 verlängert je eine Reaktorkapillare 12.

Gesteuert wird die Detektion durch ein Mikroprozessor-EDV-System, das Kenndaten über Parameter von Inhaltsstoffen, Eichung und Skalierung sowie Patienten-Informationen von einem Magnetstreifen oder Speicherchip der Chipkarte 14 entnimmt. Als Ausgabegerät kann ein Display, beispielsweise ein Großflächen-LCD-Display, verwendet werden, das die Ergebnisse der Detektion sofort anzeigt. Weiterhin sind geeignete Schnittstellen nach Industriestandard für die PC-Auswertung vorgesehen, z.B. Infrarot-Übertragung und GSM-Funkübertragung via Internet bei Einsatz im Monitoring von Risikogruppen.

Die Reaktion wird durch den Transport des in der Aufbewahrungseinrichtung 6 befindlichen Blutplasmas in die Reaktoreinrichtung 10 ausgelöst. In der Detektionseinrichtung 20 werden die Reaktionsprodukte aus den Reaktorkapillaren 12 für jede Kapillare 12 einzeln detektiert. Es kann auch eine Konzentrationsmessung oder Messung eines anderen Parameters, der einen Inhaltsstoff des Blutplasmas charakterisiert, stattfinden.

Für den Transport des Blutplasmas aus der Aufbewahrungseinrichtung 6 durch die Reaktoreinrichtung 10 in die Detektionseinrichtung 20 können aktive oder passive Systeme in Betracht kommen. Als aktives System sind Mikropumpen möglich, als passives System wiederum kapillarer Hub.

Für die Förderung ist das Flüssigkeitsreservoir 15, das eine Pufferlösung enthält, in Form eines aufgesetzten Behälters oder als eingelassene Kammer auf der Chipkarte 14 ausgebildet. Das Flüssigkeitsreservoir 15 ist mit einem Spülkanal 9 in Form einer Spülkapillare der Reaktoreinrichtung 10 verbunden. Die Spülkapillare 9 verläuft auch durch die Aufbewahrungseinrichtung 6. Sie mündet am gemeinsamen Einlass der Aufbewahrungskapillaren 4 und ist derart mit diesen verbunden. Durch ein Öffnen des Reservoirs 15 zur Umgebungsluft bei offenem Ventil 16 wird der kapillare Hub des Plasmas in die Reaktoreinrichtung 10 und Detektionseinrichtung 20 eingeleitet. Während und nach vollständiger Füllung der Reaktoreinrichtung 10 und Detektionseinrichtung 20 kann die Messung erfolgen.

Nach erfolgreicher Messung wird das System gespült, indem Druck auf das Reservoir 15 ausgeübt wird. Hierfür kann eine Außenwandung des Reservoirs, die an der Chipkartenoberfläche liegt nachgiebig sein. Durch manuellen Druck auf diese Außenwandung wird gespült. Das Reservoir 15 kann auch unter einem Überdruck stehen, der bei Öffnen des Ventils 16 für die Spülung sorgt.

Die Kapillaren 22 der Detektionseinrichtung 20 können vorteilhaft am Ausgang des Detektorenfeldes in einem Spülstrang 13 vereinigt sein, der in das Abfallreservoir 18 führt. Das Abfallreservoir 18 ist auf der Chipkarte 14 ausgebildet als aufgesetzter Behälter oder eingelassene Kammer und hat eine flüssigkeitsdichte, aber luftdurchlässige Öffnung 19 zur Umgebungsluft. Auf diese Weise wird das Blutplasma im Abfallreservoir 18 der Chipkarte gesammelt. Die Kapillaren 22 der Detektionseinrichtung 20 werden anschließend mit Pufferlösung aus dem Reservoir 15 gespült. Die Chipkarte 14 kann entnommen und entsorgt werden, beispielsweise durch Rücksendung an den Hersteller in der Rücksendebox gemäß US-PS 5,934,549.

Zur Lagerung der Detektions- und Auswerteinrichtung 20, 24 kann eine Lagerkarte eingesetzt werden, die den Detektor schützt und die ggf. ebenfalls eine Spülfunktion und eine Kalibrierfunktion zur Funktionskontrolle enthalten kann.

Weiterhin ist eine alphanumerische Eingabemöglichkeit vorgesehen, um Patientendaten eingeben zu können. Diese Daten können bei der Auswertung der Tests benutzt werden (z.B. Alter, Geschlecht, Menstruation, Gewicht, Lifestyle-Daten etc.). Die Dimensionierung des Gerätes kann durch Systemintegration auf die Größe eines handelsüblichen Funktelefons (Handy) reduziert werden. Dadurch ist neben dem Einsatz am POC eine bequeme Handhabung beim Verwender selbst gewährleistet. Die komplette Analysevorrichtung kann beim Verwender selbst vorhanden sein. Auch in diesem Fall ist die Ausbildung der Probennahme und Aufbewahrungseinrichtung als eigenständiger Vorratschip vorteilhaft für die Handhabung.

Eine umfangreiche Ausstattung von Dateneingängen- und ausgängen ermöglicht eine universelle Verwendbarkeit der Detektions- und Auswerteeinrichtung. Beispielhaft seien genannt:

Eingänge:
- Integrierte Messung des Blutdrucks, z.B. an der Fingerkuppe,
- Integrierte Messung der Körpertemperatur,
- Integrierte Kamera für Ferndiagnose,
- Anschlüsse für externe Messgeräte wie EEG, EKG, Heimtrainer, Waage, Blutdruckmessung, Thermometer und/oder Kamera.

Ausgänge:
- Funktelefon zur Übertragung der Daten zum Service-Provider und/oder Arzt/Krankenhaus,
- IR-oder Funk-Anbindung zum Computer.
- Serielle Schnittstelle zum Computer.

Die Analysevorrichtung und insbesondere der Vorratschip eignen sich zur Selbstkontrolle des Gesundheitszustands sämtlicher Bevölkerungs-Mitglieder. Durch die ermöglichte parallele Bestimmung von vielen Parametern einer Probe ist ein darüberhinausgehendes Monitoring möglich. Die Reaktoreinrichtung kann vollständig an bestimmte diagnostische Aufgaben angepasst werden, da die jeweils interessierenden Tests frei darauf kombiniert werden können. Dadurch wird ein extrem breites Einsatzspektrum eröffnet. Als Beispiele seien genannt:
- Allgemeine Gesundheits-Vorsorge. Durch ein Abonnementsystem kann der jeweilige Gesundheitsdienstleister regelmäßig Testkarten an seine Kunden versenden. Diese überwachen selbstständig wichtige Risikofaktoren (Herz-Kreislauf, Tumormarker, Urinparameter, Leberwerte etc.).
- Überwachung von Risikogruppen, Früherkennung der Krankheitsausbildung: Herz-Kreislauf, Diabetes, Krebserkrankungen, Altzheimer etc.
- Überwachung des Therapieerfolges: Chemotherapie bei Krebserkrankungen, Diabetes, Magengeschwüre etc.
- Langzeitüberwachung nach der Therapie: Sicherheit bei Rückfallgefahr.
- Monitoring bei chronischem Krankheitsverlauf zur rechtzeitigen Krisenintervention: Bluter, Astma, Allergien etc.
- Massenscreening auf Infektionen, auch in abgelegenen Gebieten ohne jegliche medizinische Infrastruktur: Aids, Malaria, Magen-Darmerkrankungen.
- Screening von Umweltmedien wie Wasser und Abwasser auf Verkeimung und Schadstoffe.
- Screening von Materialproben, beispielsweise Böden oder Hausstaub, der Rückschlüsse auf die Raumluft gestattet, auf Verkeimung und Schadstoffe.

Die Flexibilität des diagnostischen Konzepts erlaubt die Anpassung an unterschiedlichste diagnostische Aufgaben und Unterschiede in der Population.

### Bezugszeichenliste

- 1: Probennahmeeinrichtung
- 2a: Einlass
- 2: Kapillaren
- 3: Pulsator
- 4: Aufbewahrungskapillaren
- 5: Sollbruchstelle
- 6: Aufbewahrungseinrichtung
- 7: Aufbewahrungskapillaren
- 8: Vorratschip
- 8a: Ausnehmung
- 9: Spülkanal
- 10: Reaktoreinrichtung
- 11: Reaktorchip
- 12: Reaktorkapillaren
- 13: Spülstrang
- 14: Chipkarte
- 14a: Aufnahmenschacht
- 15: Flüssigkeitsreservoir
- 16: Ventil
- 17: Öffnung
- 18: Abfallreservoir
- 19: Öffnung
- 20: Detektionseinrichtung
- 21: Detektoren
- 22: Detektorkapillaren
- 23: Lichtquelle
- 24: Auswerteeinrichtung

## Patentansprüche

1. Vorrichtung zur Aufnahme und Aufbewahrung einer Substanz für eine Analyse, die Vorrichtung umfassend:
a) eine Probennahmeeinrichtung (1) mit einem Einlass (2a) zur Aufnahme der Substanz
b) und eine Aufbewahrungseinrichtung (6), die zur Aufbewahrung der Substanz Aufbewahrungskapillaren (7) aufweist, die mit der Probennahmeeinrichtung (1) verbunden sind und in die die aufgenommene fließfähige oder in einen fließfähigen Zustand versetzte Substanz durch kapillaren Hub gefördert wird,
c) wobei die Substanz in den Aufbewahrungskapillaren (7) für eine spätere Analyse bevorrated wird
d) und die Probennahmeeinrichtung (1) und die Aufbewahrungseinrichtung (6) aufoder in einem gemeinsamen Vorratschip (8) ausgebildet sind.

2. Vorrichtung nach Anspruch 1, dadurch gekennzeichnet, dass der Vorratschip (8) als Transportbehältnis zum Transport der aufgenommenen Substanz zu einem analytischen Labor verwendet wird.

3. Vorrichtung nach einem der vorhergehenden Ansprüche, dadurch gekennzeichnet, dass der Vorratschip (8) eine Sollbruchstelle (5) aufweist, an der die Aufbewahrungseinrichtung (6) von der Probennahmeeinrichtung (1) trennbar ist.

4. Vorrichtung zur Untersuchung einer Substanz, die Vorrichtung umfassend:
a) eine Probennahmeeinrichtung (1) mit einem Einlass (2a) zur Aufnahme der Substanz,
b) eine Aufbewahrungseinrichtung (6), die zur Aufbewahrung der Substanz Aufbewahrungskapillaren (7) aufweist, die mit der Probennahmeeinrichtung (1) verbunden sind und in die die aufgenommene, fließfähige oder in einen fließfähigen Zustand versetzte Substanz durch kapillaren Hub gefördert wird,
c) eine Reaktoreinrichtung (10), die als Reaktoren voneinander separate Reaktorkapillaren (12) oder eine saugfähige Materialschicht aufweist, die mit den Aufbewahrungskapillaren (7) verbindbar oder permanent verbunden sind und in die die Substanz durch kapillaren Hub oder durch einen Pumpvorgang gefördert wird,
d) wobei die Reaktoren mit wenigstens einem Reagenz belegt sind, das mit einem Inhaltstoff der Substanz ein für den Inhaltsstoff spezifisches Reaktionsprodukt bildet,
e) und eine Detektionseinrichtung (20) mit wenigstens einem Detektor (21), mit dem das Reaktionsprodukt detektierbar und vorzugsweise die Konzentration des Reaktionssprodukts messbar ist und der ein Ausgangssignal für eine Auswerteeinrichtung (24) in Abhängigkeit von der Detektion und/oder der Messung bildet.

5. Vorrichtung nach dem vorhergehenden Anspruch, dadurch gekennzeichnet, dass die Probennahmeeinrichtung (1) und die Aufbewahrungseinrichtung (6) auf oder in einem gemeinsamen Vorratschip (8) ausgebildet sind, dass die Reaktoreinrichtung (10) auf einem Träger (14) angeordnet ist und dass der Träger (14) eine Ausnehmung (8a) aufweist, in der der Vorratschip (8) aufnehmbar und die Aufbewahrungskapillaren (7) mit den Reaktorkapillaren (10) dicht verbindbar sind.

6. Vorrichtung zum Nachweis wenigstens eines Inhaltsstoffs einer Substanz, die Vorrichtung umfassend:
a) eine Probennahmeeinrichtung (1) mit einem Einlass (2) zur Aufnahme der Substanz
b) und eine Reaktoreinrichtung (9), die als Reaktoren voneinander separate Reaktorkapillaren (10) oder in Form einer saugfähigen Materialschicht aufweist, die mit der Probennahmeeinrichtung (1) verbunden sind und in die die aufgenommene, fließfähige oder in einen fließfähigen Zustand versetzte Substanz durch kapillaren Hub gefördert wird,
c) wobei die Reaktoren mit wenigstens einem Reagenz belegt sind, das mit dem Inhaltsstoff eine spezifische Farbreaktion eingeht,
d) und wobei die Probennahmeeinrichtung (1) und die Reaktoreinrichtung (9) auf einem gemeinsamen Trägerchip ausgebildet sind.

7. Vorrichtung nach einem der vorhergehenden Ansprüche, dadurch gekennzeichnet, dass die Aufbewahrungskapillaren (7) voneinander separate Mikrokanäle mit definierten Volumina sind.

8. Vorrichtung nach einem der vorhergehenden Ansprüche, dadurch gekennzeichnet, dass die Probennahmeeinrichtung (1) wenigstens eine Kapillare (2) aufweist, die den Einlass (2a) bildet oder mitbildet und mit wenigstens einer der Aufbewahrungskapillaren (7) verbunden ist.

9. Vorrichtung nach dem vorhergehenden Anspruch, dadurch gekennzeichnet, dass an einer Seitenwandung der wenigstens einen Kapillare (2) der Probennahmeeinrichtung (1) Poren einer Perforation mit solch einer Porenweite ausgebildet sind, dass in der Substanz vorhandene Körperchen nicht durch die Poren in die Kapillare (2) eindringen und an der Kapillarenaußenfläche von der eindringenden Flüssigkeit separiert werden.

10. Vorrichtung nach einem der vorhergehenden Ansprüche, dadurch gekennzeichnet, dass zwischen der Probennahmeeinrichtung (1) und den Aufbewahrungskapillaren (7) der Aufbewahrungseinrichtung (6) ein Pulsator (3) mit einer Pulsatormembran angeordnet ist, der in einem Pulsatorzustand den Einlass (2a) der Probennahmeeinrichtung (1) von den Aufbewahrungskapillaren (7) trennt.
